(19) **Europäisches Patentamt**
**European Patent Office**
**Office européen des brevets**

(11) **EP 2 538 984 B1**

(12) **EUROPEAN PATENT SPECIFICATION**

(45) Date of publication and mention
of the grant of the patent:
**21.07.2021 Bulletin 2021/29**

(51) Int Cl.:
***A61L 15/16*** *(2006.01)*     ***A61F 13/00*** *(2006.01)*
***A61L 15/22*** *(2006.01)*     ***A61L 15/44*** *(2006.01)*

(21) Application number: **11706704.1**

(22) Date of filing: **21.02.2011**

(86) International application number:
**PCT/US2011/025581**

(87) International publication number:
**WO 2011/103527 (25.08.2011 Gazette 2011/34)**

(54) **COMPRESSION DRESSING**

KOMPRESSIONSVERBAND

PANSEMENT-COMPRESSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2011 US 201113030166**
**22.02.2010 US 306629 P**

(43) Date of publication of application:
**02.01.2013 Bulletin 2013/01**

(73) Proprietor: **Milliken & Company**
**Spartanburg, South Carolina 29303 (US)**

(72) Inventors:
• **KELLER, Keith A.**
**Spartanburg**
**South Carolina 29307 (US)**
• **MACMECCAN, Robert M.**
**Greer**
**South Carolina 29650 (US)**

• **LE, Zhang**
**Spartanburg**
**South Carolina 29301 (US)**
• **CANADA, Thomas A.**
**Chesnee**
**South Carolina 29323 (US)**

(74) Representative: **Hoffmann Eitle**
**Patent- und Rechtsanwälte PartmbB**
**Arabellastraße 30**
**81925 München (DE)**

(56) References cited:
**EP-A1- 1 709 947        WO-A1-95/16416**
**US-A- 5 939 339        US-A1- 2010 030 170**
**US-A1- 2010 030 171    US-A1- 2010 030 178**
**US-B2- 6 573 419**

**Description**

## TECHNICAL FIELD

This disclosure relates to a compression dressing designed to provide moisture transport properties to a contact surface and compression therapy for patients with swelling due to various medical conditions. The compression dressing is minimally comprised of at least one layer of moisture transport fabric comprised of elastic fiber that exhibits a sufficient amount of elasticity to apply a compressive force to a body part when applied thereto. The compression dressing further provides one-way, active fluid management which can move fluid away from the skin. The compression dressing may also include at least one absorptive reservoir layer that is applied to the outer surface of the moisture transport fabric. This absorptive reservoir layer may be removed as needed without substantially disrupting the compressive force applied to a body part. The method for using the compression dressing to manage fluid movement and edema is also provided.

## BACKGROUND

Current compression dressing systems frequently employ a skin-contacting absorptive layer or substrate which is capable of absorbing wound exudate or other fluids. The use of absorbent layers beneath compression dressings is well known and has been standard practice in the medical field for some time. A number of different absorptive systems can be employed based on the particular wound or medical condition or preference of a health care professional. For example, hydrogels, hydrocolloids, super absorbent polymers, ABD pads, gauze, cotton batting and foam have been employed for this purpose.

However, over the course of a single treatment as defined by the time between initial application of the compression dressing and the need for rewrapping (frequently seven days), the absorbent material may become saturated, leading to areas of maceration and damage of healthy skin tissue. Also, the retention of fluids within the dressing for such a long period of time can lead to unpleasant odors. Current compression systems also tend to become bulky or sag as they absorb fluid.

As one example, US 5,939,339 teaches an absorbent, self-adhering elastic bandage with an integral absorptive component. The bandage is designed for use such that the absorptive layer of the bandage is in direct contact with a person's skin. The absorptive layer is integrally attached to the bandage and cannot be replaced when saturated with fluid. Rather, the entire bandage must be removed in order to replace the absorptive layer.

Similarly, US 6,573,419 discloses a compression dressing comprising a self-adhering elastic bandage strip designed for exerting a compressive force when wrapped around a body part. The compressive force is sufficient to hold the compression dressing in place for a period of time to provide a therapeutic effect to the patient. An absorbent pad is affixed to the skin-contact surface of the bandage. Thus, the absorbent pad cannot be replaced without removing the entire bandage and disrupting the compressive force previously applied to the body part.

EP-A-1 709 947 discloses a compression bandaging system comprising (a) an inner skin facing, elongated, elastic bandage comprising (i) an elongated, elastic substrate, and (ii) an elongated layer of foam being affixed to a face of said substrate and extending ≥ 33% across the face of substrate in transverse direction and ≥ 67% across the face of substrate in longitudinal direction; and (b) an outer, elongated, self-adhering elastic bandage having a compressive force when extended; wherein, in use, said foam layer of the inner bandage faces the skin and the outer bandage overlies the inner bandage.

US-A-2010/030170 and US-A-2010/030171 relate to a composite article comprising (a) a fluid transport layer having a first and a second surface, the first surface providing a fluid contacting surface having a first surface energy, and the second surface having a second surface energy, and (b) a fluid retentive layer having a first and a second surface, the first surface having a third surface energy, and the fluid retentive layer being positioned so that its first surface is adjacent to the second surface of the fluid transport layer; and wherein the third surface energy is greater than the second surface energy. US-A-2010/030178 addresses a similar composite article wherein in the fluid transport layer the second surface energy is greater than the first surface energy, and the second layer (b) is a hydrophobic layer applied to the first surface of the fluid transport layer and having a third surface energy lower than the first surface energy, and the layer (b) comprises a plurality of discontinuities at least a portion of which have a dimension sufficient to permit the passage of fluid through the layer (b) and into the layer (a).

US 5,939,339 describes a compressive dressing comprising (a) a conformable, porous, self-adhering elastomeric substrate which does not adhere to clothing, hair or skin and which has a compressive force when extended that is sufficient to hold the dressing in place for a period of time to provide a therapeutic effect to the wound; and (b) an absorbent layer which is flexibly bonded to the self-adhering substrate and is offset to one end of the self adhering substrate.

US 6,573,419 provides a compression dressing, comprising (a) an elongated, self-adhering elastic bandage strip; and (b) an absorbent pad being of substantially lesser length than the self-adhering elastic bandage strip and being affixed to a terminal end of the self-adhering elastic bandage strip; the elastic bandage strip being designed, and of sufficient length, such that the absorbent pad may be applied to a wound on a body part, and the remainder of said strip or portion thereof, may be wrapped around the absorbent pad and the body part to hold the compression dressing in place and to exert a compressive force on the wound for a period of time to permit a therapeutic effect to the wound.

Accordingly, there is a need in the medical field for a compression dressing which allows for an absorptive layer to be replaceable without disrupting the compressive force applied to a body part and, at the same time, manages the movement of fluid away from the skin surface which results in less surface wetness at the skin-to-compression dressing interface. Less surface wetness results in less skin maceration and/or better skin health. Additionally, swelling of the absorptive reservoir layer, which may occur as it absorbs fluids, would not detrimentally affect the overall shape of the compression system since the absorptive reservoir layer is located exterior to the moisture transport layer.

Thus, the present invention provides an improvement over the current compression dressing systems by allowing the patient and/or medical professional to replace the absorptive portion of a compression dressing system without substantially disrupting the compressive force applied to the body part wrapped with the dressing. In some cases, by allowing the patient to easily remove the outer absorptive reservoir layer when needed, without disrupting the moisture transport layer, time and expense associated with medical care for replacing soaked dressings may be desirably reduced. In some cases, as needed, more frequent changes of the absorptive media may occur, which may be beneficial for healing and/or for skin health at the skin-to-compression dressing interface. More frequent dressing changes may also lead to less odor build-up at the skin-to-compression dressing interface. For these reasons and others that will be described herein, the present compression dressing represents a useful advance over the prior art.

## SUMMARY

Provided herein is a compression dressing which

(i) is at least 5.1 cm (2 inches) wide and 91.4 cm (36 inches) in length;
(ii) provides one-way, active fluid management of fluid away from the skin and into the absorptive reservoir layer,
(iii) comprises the following sequential layers:

(a) optionally, a skin-contacting layer comprised of fabric, foam, alginate, hydrogel, hydrocolloid, polymeric film, medicinal agents, and combinations thereof,
(b) at least one layer of moisture transport fabric which

- is comprised of elastic fiber,
- exhibits a sufficient amount of elasticity to apply a compressive force to a body part when applied thereto;

- is a jersey knit fabric comprised of 100% synthetic fiber which is a blend of 40-80% nylon fiber, 10-50% polyester fiber and 5-25% spandex fiber, and
- is comprised of hydrophobic fiber on the skin-facing surface of the fabric and hydrophilic fiber on the non skin-facing surface of the fabric,

(c) at least one absorptive reservoir layer selected from textile substrates, foam, hydrogel, hydrocolloid, superabsorbent polymers, silica gel, water swelling polymers, polysaccharides, proteinaceous materials, and combinations thereof; and
(d) optionally, at least one layer of elastic fabric comprising the outermost layer of the compression dressing; and

(iv) exhibits a tensile strength of 0.89-25.67 kg/m width (0.05-2.0 Ibf/inch width) at 50% elongation, determined using a MTS Sintech 10/G mechanical tester with an MTS 25kN load cell and stretching at 20.3 cm/min (8 inches/min).

Preferred embodiments of the present compression dressing are as defined in the appended dependent claims and/or in the following detailed description.

## BRIEF DESCRIPTION OF DRAWINGS

FIG. 1 is a perspective view of one embodiment of the present compression dressing.
FIG. 1A is an exploded, perspective view of the compression dressing depicted in **FIG. 1.**
FIG. 2 is a plan view of a laid-in fabric suitable for use as the moisture transport fabric layer of the present compression dressing.

FIG. 3 is an exploded, perspective view of yet another embodiment of the present compression dressing.

FIG. 4 is an illustration of the present compression dressing, in association with a body part.

FIG. 5A is a perspective view of an absorptive reservoir layer suitable for use in the present compression dressing.

FIG. 5B is a perspective view of an absorptive reservoir layer suitable for use in the compression dressing according to the invention.

FIG. 5C is an exploded, perspective view of a further embodiment of the present compression dressing.

FIG. 6 is a line graph illustrating the relationship between pressure, elongation, and load per inch for the present compression dressing.

## DETAILED DESCRIPTION

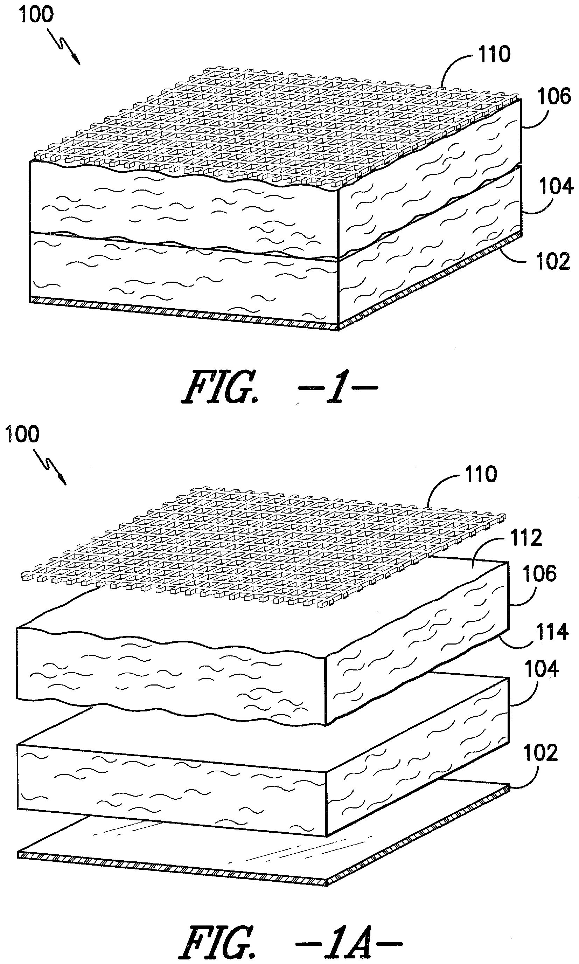

FIGS. 1-5 are representative depictions of the various components which may comprise the present compression dressing. In the Figures, the bottom layer represents the layer of the compression dressing which is located the furthest away from the intended contact surface, while the top layer represents the layer of the compression dressing which is intended to come in contact with a surface (i.e. the skin-contact layer). Thus, the bottom layer depicted in the Figures is the outermost layer of the compression dressing, while the top layer is the innermost layer of the compression dressing.

At various stages of venous diseases, such as varicose veins, chronic venous insufficiency, venous stasis, and venous reflux, different levels of compression of 8-60 mm Hg are clinically indicated. According to Laplace's law, sub-bandage pressure is directly proportional to bandage tension, the number of layers applied, and inversely proportional to the radius of curvature of the limb to which it is applied. Laplace's law can be expressed as following equation,

$$P_{Compression} = \frac{Fn}{w\,R} \qquad [1.2]$$

wherein F is the tension of bandage, n is the number of layers applied, w is the bandage width, and R is the leg radius. Assuming that a bandage of standard width is applied in the form of a spiral with a 50% overlap to a patient with a leg (or other body part) of relatively fixed dimensions, bandage tension is the only variable in this equation, which illustrates the importance of this factor in the determination of sub-bandage pressure. The ability of a bandage to sustain a specific degree of tension is directly related with its elastomeric properties, as determined by fabric construction and finishing. The ability of a bandage to increase in length in response to an applied force is described as its extensibility, also known as percent strain or percent elongation.

As such, the Force of the bandage divided by the width of the bandage (F/w) is significant in applying the correct amount of pressure to the wrapped body part. This force is a function of the bandage extension or elongation and is tested using a tensile tester.

FIG. 1 depicts a four-layer compression dressing 100 in which the outermost layer is an elastic fabric layer 102. The presence of elastic fabric layer 102 in the compression dressing 100 is optional. As used herein, the term "elastic" or "elastomeric" is intended to encompass any material that provides the layer with the ability to be stretched and then return to dimensions that are substantially the same as its original dimensions. For example, elastic fabric layer 102 may be comprised of elastomeric fibers, such as a Spandex yarn (i.e., a manufactured fiber in which the fiber-forming substance is a long chain synthetic polymer composed of at least 85% of a segmented polyurethane). The elastic fabric layer 102 may have a woven, knit or nonwoven construction that is comprised of natural fiber, synthetic fiber, or blends thereof, as further described herein. An adhesive material may also be included in the layer. The elastic fabric layer 102 may be provided in the form of an elastic stocking.

The next layer in the multi-layer compression dressing 100 is an absorptive reservoir layer 104 that exhibits high absorptive properties and acts as a fluid reservoir. Absorptive reservoir layer 104 may be comprised of any type of material capable of providing absorbent properties to the compression dressing 100 and being selected from textile substrates (e.g., woven or knit fabrics and nonwovens), foam, hydrogel, hydrocolloid, superabsorbent polymers, silica gel, water swelling polymers, polysaccharides (e.g., chitosan, carboxymethylcellulose, hydroxylmethylcellulose, hyaluronic acid, alginates and pectin) and proteinaceous materials (such as glycoproteins and gelatins) and combinations thereof.

The absorptive reservoir layer 104 of the compression dressing can exhibit any suitable absorptive capacity. For example, absorptive reservoir layer 104 may exhibit a fluid absorption of ≥ 100 wt% based on the weight of the absorptive reservoir layer. In a specific embodiment, the absorptive reservoir layer may exhibit a fluid absorption of ≥ 200 wt%, ≥ 300 wt%, ≥400 wt%, ≥ 500 wt%, ≥ 600 wt%, ≥ 700 wt%, ≥ 800 wt%, ≥ 900 wt% or ≥ 1000 wt% based on the weight of the absorptive reservoir layer. The absorptive capacity of the absorptive reservoir layer may be measured by any suitable means. For example, the absorptive capacity of the absorptive reservoir layer may be determined by comparing the weight of the layer before and after immersing the absorptive reservoir layer in an aqueous solution of phosphate-buffered saline that contains 0.9 wt% sodium chloride at 37°C until saturation is achieved.

The next layer in the compression dressing **100** is a moisture transport fabric layer **106** that exhibits the ability to move fluid, uni-directionally, away from the fluid source and into the absorptive reservoir layer **104.** Moisture transport fabric layer **106** is comprised of any type of textile substrate capable of providing high wicking properties to the compression dressing **100.**

Layers **104** and **106** may be independently comprised of textile substrates, such as fabrics, having a woven, nonwoven, or knit construction. Suitable knit textiles include, but are not limited to, weft-knit textiles, such as flat-knit textiles and circular-knit textiles. Fiber types comprising the textile substrate include synthetic fibers, natural fibers, and mixtures thereof. Synthetic fibers include, for example, polyester, acrylic, polyamide, polyolefin, polyaramid, polyurethane, regenerated cellulose (i.e., rayon), cellulose derivatives, and blends thereof. The term "polyamide" is intended to describe any long-chain polymer having recurring amide groups as an integral part of the polymer chain. Examples of polyamides include nylon 6; nylon 6,6; nylon 1,1; and nylon 6,10. The term "polyester" is intended to describe any long-chain polymer having recurring ester groups. Examples of polyesters include aromatic polyesters, such as polyethylene terephthalate (PET), polybutylene terephthalate (PBT), polytrimethylene terephthalate (PTT), and polytriphenylene terephthalate, and aliphatic polyesters, such as polylactic acid (PLA). "Polyolefin" includes, for example, polypropylene, polyethylene, and combinations thereof. "Polyaramid" includes, for example, poly-p-phenyleneteraphthalamid (i.e., Kevlar®), poly-m-phenyleneteraphthalamid (i.e., Nomex®), and combinations thereof. Natural fibers include, for example, wool, cotton, flax, bamboo, and blends thereof.

The textile substrate may be formed from fibers or yarns of any size, including microdenier fibers and yarns (fibers or yarns having less than one denier per filament). The fibers or yarns may have deniers of < 1 to 2000 denier per filament, more preferably < 1 to 500 denier per filament, even more preferably < 1 to 300 denier per filament.

Furthermore, the textile substrate may be partially or wholly comprised of multi-component or bicomponent fibers or yarns, which may be splittable, or which have been partially or fully split, along their length by chemical or mechanical action. The fabric may be comprised of fibers such as staple fiber, filament fiber, spun fiber, or combinations thereof.

The textile substrate may optionally be colored by a variety of dyeing techniques, such as jet dyeing, vat dyeing, thermosol dyeing, pad dyeing, transfer printing, screen printing, flexigraphic printing or any other technique that is common in the art for comparable textile products.

A skin-contacting layer **110** is the next layer in the compression dressing **100** and forms the innermost layer of the compression dressing **100.** The presence of skin-contacting layer **110** in the compression dressing **100** is optional. The skin-contacting layer **110** may provide anti-stick properties to the compression dressing such that its presence prevents the compression dressing from sticking to the skin and/or wound site. The skin-contacting layer **110** may also contain medicinal agents which aid in wound healing. Exemplary medicinal agents include, without limitation creams, ointments, balms, biologic scaffolds (e.g. collagen, modified collagen, extra cellular matrices and porcine sub-intestinal mucosa), skin grafts, and combinations thereof. Suitable materials for use as the skin-contacting layer **110** are foam, alginate, hydrogel, hydrocolloid, polymeric film (e.g. perforated polymeric film), and combinations thereof. Preferably, the optional skin contact layer **110** will not substantially impede or restrict the movement of moisture (e.g. wound fluid and sweat) into the successive layers of the compression dressing **100.** In one embodiment, the skin-contacting layer **110** may be attached to the moisture fabric transport layer **106.** For example, layer **110** may be laminated to layer **106** prior to application to a body part.

As depicted in **FIG. 1A,** the moisture transport fabric layer **106** comprises a first surface **112** and a second surface **114.** The first surface **112** of the moisture transport fabric layer **106** has a first surface energy, and the second surface **114** of the moisture transport fabric layer **106** has a second surface energy. As utilized herein, the term "surface energy" refers to the excess energy at the surface of a material compared to the bulk of the material (e.g., the interior portions of the material) and is usually expressed in terms of milliJoules per square meter ($mJ/m^2$). The surface energy quantifies the disruption of intermolecular bonds that occurs when a surface is created. The surface energy can be measured by several means including, for example, the Fowkes method. In this method, two reference liquids are used to first measure the dispersive component and the polar component of the material's surface energy. The surface energy of the material is then calculated from the measured dispersive and polar components. In general, a surface having a higher surface energy will exhibit a higher affinity for aqueous fluids, such as perspiration or wound exudate.

In certain embodiments, the surface energy of the first surface **112** of the moisture transport fabric layer **106** and the surface energy of the second surface **114** of the moisture transport fabric layer **106** can be substantially the same. In a specific embodiment, the surface energy of the second surface **114** of the moisture transport fabric layer **106** is greater than the surface energy of the first surface **112** of the moisture transport fabric layer **106.** This difference in surface energies between the two surfaces means that the second surface **114** of the moisture transport fabric layer **106** exhibits a greater affinity for aqueous fluids (e.g. perspiration or wound exudates) than the first surface **112** of the moisture transport fabric layer **106.** Thus, any aqueous fluids absorbed by the moisture transport fabric layer **106** will be transported or pumped from the first surface **112** to the second surface **114** of the moisture transport fabric layer **106.** This active transportation or pumping of the fluids ensures that excess moisture does not accumulate at the interface of moisture transport fabric layer **106** and a moisture or fluid exuding surface, such as the skin or an exuding wound.

When the fluid transport layer comprises first and second surfaces having different surface energies, the difference between the two surface energies can be of any suitable magnitude. In a specific embodiment, the surface energy of the second surface **114** of the moisture transport fabric layer **106** can be ≥ 101% of the surface energy of the first surface **112** of the moisture transport fabric layer **106.** In more specific embodiments, the surface energy of the second surface **114** can be ≥ 102%, ≥ 103%, or ≥ 104% of the surface energy of the first surface **112.**

In addition to differences in surface energy, differences in capillarity can also yield desired fluid transport properties. As utilized herein, the term "capillarity" refers to the ability of a material to retain or expel fluid. In this regard, the capillarity of a material is determined by the pore size of the material as well as the contact angle with the fluid in question. Capillarity is measured by the ability of a material to either resist pressure when submerged to a given depth in an aqueous fluid or to draw aqueous fluid to a given height. Thus, the capillarity is the height of fluid drawn up (if positive) or height immersed (if negative) times the fluid density. The capillarity is defined by the following equation:

$$C = \frac{2\gamma\cos\theta}{gr}$$

- $\gamma$ is the liquid-air surface tension (energy/area)
- $\theta$ is the contact angle
- $g$ is acceleration due to gravity (length/time$^2$)
- $r$ is pore radius

In this regard, the effective pore radius for a fabric with multiple pore sizes is generally dependent upon the character of the fabric. For materials that draw fluid inwardly, the effective radius tends to be dominated by the smaller pore sizes. For materials that expel fluid, the effective radius tends to be dominated by the larger pore sizes. As will be appreciated, in general, a positive capillarity will be associated with a generally fluid absorptive material while a negative capillarity will be associated with a material that expels fluid. As will be recognized by those of skill in the art, capillarity levels may be measured directly by using known techniques including wetting tests such as wicking and dewetting tests such as liquid extrusion porosimetry or porometry.

In one example, a jersey knit fabric comprised of predominantly polyester fiber on one side and nylon fiber on the opposite side may be utilized. The jersey knit fabric may be arranged such that the polyester side of the fabric contacts the moisture exuding surface (e.g. skin and/or wound), and the nylon side of the fabric contacts the absorptive reservoir layer **104.** It may be generally known to those skilled in the art that a knit polyester fabric tends to be hydrophobic, slow to absorb liquids, and generally exhibits little or no wicking of moisture. Since polyester is hydrophobic in nature, conventional wisdom would lead one to choose a hydrophilic natural fiber, such as cotton, or a hydrophilic synthetic fiber, such as nylon, as the skin contacting side of the compression dressing. However, by placing a hydrophobic polyester containing surface toward the skin and a hydrophilic nylon containing surface away from the skin, a unique one-way, directional flow of fluid away from the skin was achieved.

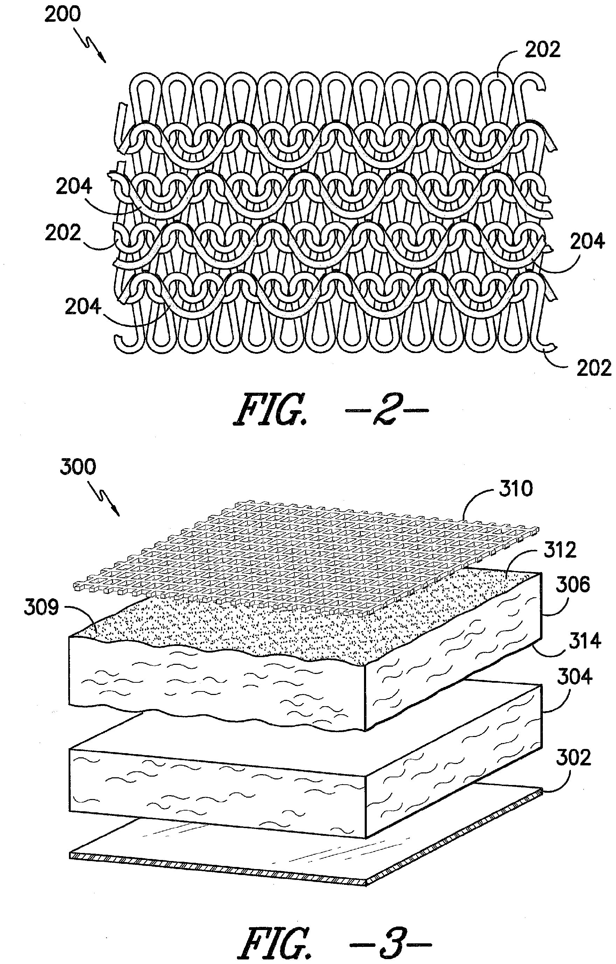

As depicted in **FIG. 2,** the moisture transport fabric layer **200** can be a laid-in fabric comprising one or more yarns **202** providing the knit structure of the fabric and one or more effect yarns **204** tucked into the fabric structure. In order to provide two surfaces having different surface energies, the moisture transport fabric layer **200** depicted in **FIG. 2** is a jersey knit fabric in which the effect yarn(s) **204** are incorporated into the fabric structure so that the effect yarn(s) **204** are predominantly present on the technical back of the fabric structure. In other words, the effect yarn(s) **204** are incorporated in the fabric structure so that most of the effect yarn(s) **204** (e.g., most of the surface area of the effect yarn(s)) is present on the technical back of the fabric structure. Such a construction results in a fabric in which the technical face of the fabric is predominantly one type of yarn **202,** and the technical back presents a higher proportion of the effect yarn(s) **204.** Thus, when the yarn **202** and the effect yarn **204** have different surface energies or one is more hydrophilic than the other, the resulting fabric will exhibit a different surface energy on each of its major surfaces.

In the moisture transport fabric layer **200** as depicted in **FIG. 2,** the yarn(s) **202** are more hydrophilic than the effect yarn(s) **204.** For example, the yarn(s) **202** can be polyamide yarns (e.g., nylon yarns), and the effect yarn(s) **204** can be polyester yarns. The moisture transport fabric layer **200** is comprised of 100% synthetic fiber. More specifically, the moisture transport fabric layer **200** is comprised of synthetic fiber that is a blend of 10-50% polyester fiber, 40-80% nylon fiber, and 5-25% spandex fiber. Such a moisture transport fabric layer **200** provides a layer in which the technical face of the fabric exhibits a higher surface energy than the technical back of the fabric. Thus, when utilized as the moisture transport fabric layer of the compression dressing depicted in **FIG. 1** and **FIG. 1A,** such a fabric (i.e., fabric **200** depicted in **FIG. 2**) is disposed so that the technical back of the fabric forms the first surface **112** of the moisture transport fabric layer **106** and the technical face of the fabric forms the second surface **114** of the moisture transport fabric layer **106.**

An optional feature of the compression dressing of the present invention is that it may contain a topical coating of an antimicrobial agent, such as silver. It is known that placing surface-available silver in contact with a wound allows the silver to become absorbed by undesirable bacteria and fungi that grow and prosper in the warm, moist environment of the wound site. Once absorbed, the silver ions kill microbes, resulting in treatment of infected wounds or the prevention of infection in at-risk wounds.

**FIG. 3** depicts a compression dressing **300** in which an antimicrobial agent **309** has been applied to the surface of the moisture transport fabric layer **306.** Antimicrobial agent **309** may be applied to either surface **312** or **314** of moisture transport fabric layer **306,** or it may be applied to both surfaces **312** and **314** of the moisture transport fabric layer **306.** As in **FIG. 1** and **FIG. 1A,** the outermost layer of the compression dressing **300** is a layer of elastic fabric **302.** The next layer in the compression dressing **300** is an absorptive reservoir layer **304** and may be comprised of any suitable material as described herein for absorptive reservoir layer **104.** The moisture transport fluid fabric layer **306** that contains an antimicrobial agent is the next layer and may be comprised of any suitable material as described herein for moisture transport fabric layer **306.**

The antimicrobial agent may include at least one silver-ion containing compound selected from silver ion exchange materials (e.g. silver zirconium phosphates, silver calcium phosphates and silver zeolites), silver particles (e.g. silver metal, nanosilver, colloidal silver), silver salts (e.g. AgCl, $Ag_2CO_3$), silver glass, and mixtures thereof. One preferred silver ion-containing compound is an antimicrobial silver sodium hydrogen zirconium phosphate compound available from Milliken & Company of Spartanburg, South Carolina, sold under the tradename AlphaSan® silver antimicrobial. Other potentially preferred silver-containing antimicrobials suitable for use herein-including silver zeolites, such as a silver ion-loaded zeolite available from Sinanen Co., Ltd. of Tokyo, Japan under the tradename Zeomic®, and silver glass, such as those available from Ishizuka Glass Co., Ltd. of Japan under the tradename Ionpure®-may be utilized either in addition to, or as a substitute for, the preferred species listed above. Various combinations of these silver-containing materials may also be utilized.

Total add-on levels of silver to the target substrate may be 5-20,000 ppm, more preferably 20-20,000 ppm, and even more preferably 200-20,000 ppm. Although these ranges are provided, an upper boundary limit of silver add-on levels to the target substrate may be limited only by consideration of the manufacturing economics of the product and by the potential to irritate a sensitive wound site, such that one would want to avoid excessive silver levels.

Preferably, the amount of silver-ion containing antimicrobial agent added to the moisture transport fabric layer should be such that moisture transport fabric layer is non-electrically conductive. "Non-electrically conductive" is defined as having a resistance in $\Omega/cm^2$ of fabric ($\Omega/in^2$ of fabric)of > 64,516 $\Omega$ (> 10,000 $\Omega$), preferably > 645,160 $\Omega$ (> 100,000 $\Omega$) and most preferably > 6.4516 x $10^9$ $\Omega$ (> 1 x $10^9$ $\Omega$), when measured in accordance with AATCC Test Method 76-1978.

The antimicrobial agent applied to the moisture transport fabric layer may also comprise non-silver compounds. These include, for example, compounds that contain copper, zinc, iodine, triclosan, polyhexamethylene biguanide (PHMB), N-halamines, chlorhexidine, quaternary ammonium complexes, and mixtures thereof, as well as common antibiotic pharmaceutical compounds. It is also contemplated that non-silver ion containing compounds may be combined with silver-ion containing compounds to form the antimicrobial agent.

Generally, the antimicrobial agent is added to the compression dressing in an amount of0.01-60% by total weight of the particular finish composition; more preferably, 0.05-40%; and most preferably 0.1-30%. The antimicrobial finish may include other components such as binder materials, wetting agents, odor absorbing agents, leveling agents, adherents and thickeners.

The inclusion of a binder material with the antimicrobial agent has been found useful in preventing the antimicrobial agent from flaking onto and/or into the wound that is being treated. Preferably, this component is a polyurethane-based binder material, although a wide variety of cationic, anionic, and non-ionic binders may also be used, either alone or in combination. Specific examples include nonionic permanent press binders (e.g., cross-linked adhesion promotion compounds, including, without limitation, cross-linked imidazolidinones available from Sequa under the tradename Permafresh®) or slightly anionic binders (including, without limitation, acrylics such as Rhoplex® TR3082 from Rohm & Haas). Other nonionics and slightly anionics are also suitable, including e.g. melamine formaldehyde, melamine urea and ethoxylated polyesters (such as Lubril QCX™, available from Rhodia). Preferably, the binder material is biocompatible such that it does not cause negative reactions in the wound. In essence, the binder materials assist in adhering the antimicrobial agent to the surface of the target substrate, such as fibers or fabrics, without negatively affecting the release of silver ions to the wound.

One exemplary acceptable method of providing an antimicrobial silver-treated fabric surface includes the application of a silver ion-containing compound and polyurethane-based binder resin from a bath mixture. This mixture of antimicrobial compound and binder resin may be applied through any technique as is known in the art, including e.g. spraying, dipping, padding, foaming and printing. By using one or more of these application techniques, a fabric may be treated with the antimicrobial compound and binder resin on only one side of the fabric, or it may be treated on both sides of the fabric. Methods of topically applying a silver-based antimicrobial finish to textile substrates are described, for example, in US 6,584,668; US 6,821,936; US 6,946,433; US-A-2004/0106340 and US-A-2004/0106341.

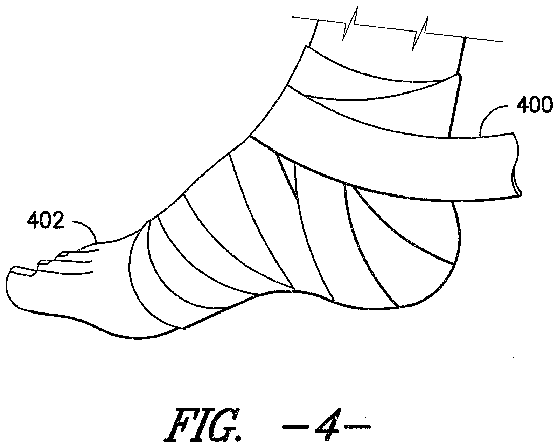

In **FIG. 4,** one embodiment of the present invention is illustrated. The compression dressing **400** is in association with a body part **402** (e.g. a foot). The compression dressing **400** is applied to the body part **402** by successively wrapping a fixed length of compression dressing circumferentially around the body part **402** such that each wrapping at least partially overlaps a portion of a previously applied wrapping. Preferably, the amount of overlap that occurs as the compression dressing is wrapped around a body part is 30-75%, more preferably 40-60%, and even more preferably 50%.

The production process for making the present compression dressing may include bringing together the various layers of the pad, such as from rolled goods; cutting the layers into the desired shape; and combining the layers together for use, such as by sewing or thermal sealing of the edges, to yield a finished, properly dimensionalized product. Alternatively, the layers could be laminated together at wide widths, as the layers are taken from the rolled goods, and then the laminated layers may be cut into the properly dimensionalized product. For instance, the compression dressing may be characterized by having dimensions that are $\geq$ 5.08 cm, $\geq$ 7.62 cm, $\geq$ 10.16 cm, or $\geq$ 12.7 cm ($\geq$ 2 inches, $\geq$ 3 inches, $\geq$ 4 inches, or $\geq$ 5 inches) in width. Further, the compression dressing may be characterized by having dimensions that are $\geq$ 91.4 cm ($\geq$ 36 inches) or $\geq$ 121.9 cm ($\geq$ 48 inches) in length. These dimensions may be mixed and matched in any manner to achieve a compression dressing that is characterized by having dimensions of width and length most suitable for treating a given medical condition. It may be preferable that the compression dressing is characterized by having dimensions that are $\geq$ 5.08 cm ($\geq$ 2 inches) wide and $\geq$ 91.4 cm ($\geq$ 36 inches) in length. The compression dressing may further be characterized by having dimensions that are $\geq$ 10.16 cm ($\geq$ 4 inches) wide and $\geq$ 121.9 cm ($\geq$ 48 inches) in length. It is contemplated that each finished product will be individually packaged in standard medical packaging material and then sterilized, such as by gamma irradiation.

Each of the layers comprising the compression dressing of the present invention is arranged substantially coextensive with each another. The layers of the compression dressing may be affixed to each other, or joined together, by any conventional process. For example, the layers may be joined by using adhesives, by sewing the layers together, via thermal sealing of the edges, by spot lamination, by ultrasonic lamination, and combinations thereof.

The compression dressing may also be secured in place during use (e.g. in association with a body part) by a variety of attachment means. Suitable attachment means include any implement that is capable of securing the compression dressing in place, when in association with a body part. Exemplary attachment means include clips, pins, adhesives (e.g. tape and liquid adhesives), loop and hook closures, zippers, buttons, snaps, self-adhering coatings, and combinations thereof.

The compression dressing may also be utilized as a drug delivery apparatus by incorporating certain compounds into one or more layers of the compression dressing which may be released for use in or on a patient. For instance, these compounds may include e.g. antibiotics, pain relievers, peptides, growth factors, anti-inflammatory agents, enzymatic or other debriding agents, and mixtures thereof.

Other additives may be present on and/or within the fabric or yarn comprising the compression dressing, including e.g. antistatic agents, optical brightening compounds, opacifiers (such as titanium dioxide), nucleating agents, antioxidants, UV stabilizers, fillers, permanent press finishes, softeners, lubricants, curing accelerators and adhesives. The layers comprising the compression dressing may also be coated or printed or otherwise aesthetically modified. Printing may be achieved, for example, by screenprinting or flexographic printing techniques.

The compression dressing itself may further include additional additives as needed for desirable end-use attributes. One exemplary additive includes superabsorbing polymers such as polyacrylic acid, polyacrylamide-containing polymers, polyvinyl alcohol, and mixtures thereof.

The compression dressing may be of any thickness, depending on the construction of the fabric and the desired level of padding and/or absorbency needed. It may, however, be preferred that the thickness of the compression dressing is 0.16-5.08 cm (0.0625-2 inches), more preferably 0.32-1.27 cm (0.125-0.5 inches).

An additional advantageous feature of the compression dressing of the present invention is its ability to substantially maintain its original color when effective amounts of a silver-ion containing antimicrobial agent are included in the dressing. The elimination of color normally associated with the inclusion of silver-based antimicrobials is highly beneficial and desirable. The compression dressing (preferably, white-colored) allows users thereof and their health care providers to monitor the exudates from a wound. Further, the compression dressing generally exhibits long-term color stability (that is, the color does not change significantly over time while in production, transit, or storage). Finally, because the compression dressing is not discolored by the addition of the silver-ion containing antimicrobial agent, a variety of substrate colors may be utilized. Colored substrates may be achieved by dyeing or coloring to any desired shade or hue with any type of colorant, such as, for example, pigments, dyes, tints and polymeric colorants.

Methods for use of the compression dressing of the present invention in association with a body part dressing include the steps of (a) applying a skin-contacting layer onto or around a body part; (b) circumferentially and successively wrapping at least one layer of moisture transport fabric comprised of elastic fiber around the body part, wherein the fabric has a first skin-facing surface that is primarily hydrophobic in nature and a second non skin-facing surface that is primarily hydrophilic in nature; (c) successively wrapping at least one absorptive reservoir layer onto or around the body part; and (d) circumferentially and successively wrapping at least one layer of elastic fabric around the body part. Steps "a" and "d" are optional. The layers of the compression dressing should be ≥ 5.08 cm (≥ 2 inches) wide and ≥ 91.4 cm (≥ 36 inches) in length. Additionally, the compression dressing should provide one-way, active fluid management of fluid away from the skin and into the absorptive reservoir layer. The layers comprising the compression dressing are preferably applied sequentially as described above. This method may be ideal for use in reducing edema in a body part. Each layer present in the compression dressing may be wrapped circumferentially and/or successively around the body part as desired for the specific medical condition to be treated.

The amount of compressive force provided by the compression dressing, when applied to a body part, is directly related to the resistive force of the material at a given strain and can be expressed in units of kg/m width of material (pounds force per inch width (lbf/in) of material). Typically, compression layers are applied at approximately 50% strain. In order to achieve acceptable levels of compression, the material exhibits a resistive force of 0.89-35.67 kg/m (0.05-2.0 lbf/in) width, preferably 1.25-17.83 kg/m (0.07-1.0 lbf/in) width, and more preferably 1.78-10.70 kg/m (0.1-0.6 lbf/in) width, at 50% strain. However, the exact amount of compressive force applied to a body part by the dressing of the present invention may depend on the body part being wrapped, the specific medical condition being treated, and application technique.

Additionally, when the absorptive reservoir layer is removed from the compression dressing, the moisture transport layer will preferably continue to provide a compressive force to the body part. Thus, removal of the absorptive reservoir layer can occur without substantially disrupting the compressive force provided by the compression dressing. As used herein, "without substantially disrupting the compressive force" may include a change in compressive force of 0-10%, more preferably 0-5%. Accordingly, when the absorptive reservoir layer is removed, due to fluid saturation or other reasons that it may need replacing, the remaining layers of the compressive dressing will continue to provide ≥ 90% of the compressive force to the body part that it had provided prior to removal of the absorptive reservoir layer.

In one embodiment, the compression dressing of the present invention comprises at least one layer of moisture transport fabric comprised of elastic fiber, wherein the moisture transport fabric exhibits a sufficient amount of elasticity to apply a compressive force to a body part when applied thereto; wherein the moisture transport fabric is wrapped circumferentially and successively around a body part; wherein the compression dressing is ≥ 5.08 cm (≥ 2 inches) wide and ≥ 91.4 cm (≥ 36 inches) in length; and wherein the compression dressing provides one-way, active fluid management of fluid away from the body part and into the moisture transport fabric.

The moisture transport fabric of the compression dressing may be further characterized by having a first skin-facing surface which is primarily hydrophobic in nature and a second non skin-facing surface which is primarily hydrophilic in nature, wherein the hydrophobic and hydrophilic surfaces partially overlap each other when wrapped circumferentially and successively around a body part such that fluid transport occurs between sequential layers of hydrophilic and hydrophobic fabric.

In one embodiment, the skin-contacting layer may be attached to the moisture fabric transport layer. For example, the skin-contacting layer may be laminated to the moisture fabric transport layer prior to application to a body part. In this instance, the combined layers may then be wrapped circumferentially and successively around a body part.

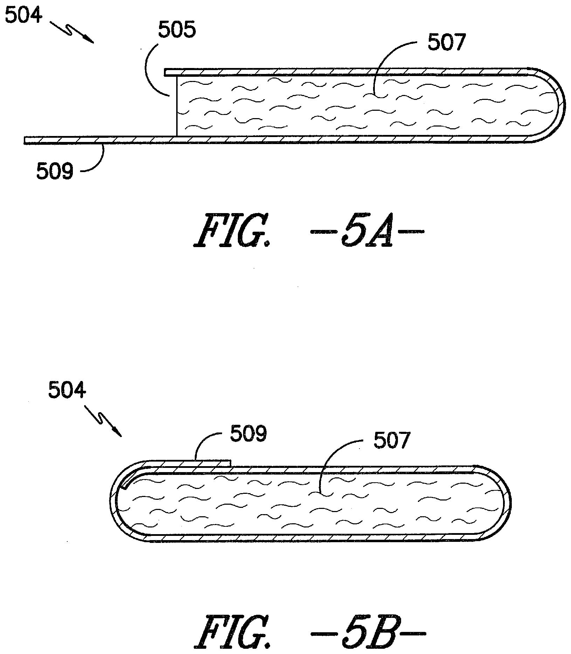

FIGS. 5A-5C illustrates further potential embodiments of the compression dressing 500 of the present invention. FIG. 5A depicts the absorptive reservoir layer 504 in a pouch configuration having a closure means 509. As used herein, the phrase "pouch configuration" is intended to describe a shape similar to a typical tea bag, wherein a bag-like structure is provided and absorptive material may be placed within the bag-like structure through an opening in the bag-like structure. FIG 5A. further illustrates that absorptive material 507 may be added to the absorptive reservoir layer 504 through an opening 505 in the absorptive reservoir layer 504.

FIG. 5B illustrates that after adding absorptive material 507 to the absorptive reservoir layer 504 (an absorptive material-containing bag-like structure), the opening 505 may then be closed by any closure means 509 The presence of the closure means 509 will prevent the absorptive material 507 from undesirably coming out of the absorptive reservoir layer 504. Any closure means 509 that acts to prevent the absorptive material 507 from undesirably coming out of the absorptive reservoir layer 504 is suitable. For instance, the closure means 509 may be in the configuration of a re-closable flap comprising the same material of the absorptive reservoir layer 504. The re-closable flap may be opened and closed multiple times. Alternatively, the closure means 509 may be in the form of a pressure sensitive adhesive that may or may not be opened and closed multiple times. The use of a re-closable closure means allows for the absorptive reservoir layer to be opened so that any saturated absorptive material can be removed from the bag-like structure and replaced with new absorptive material. The closure means may then be re-secured in a closed position.

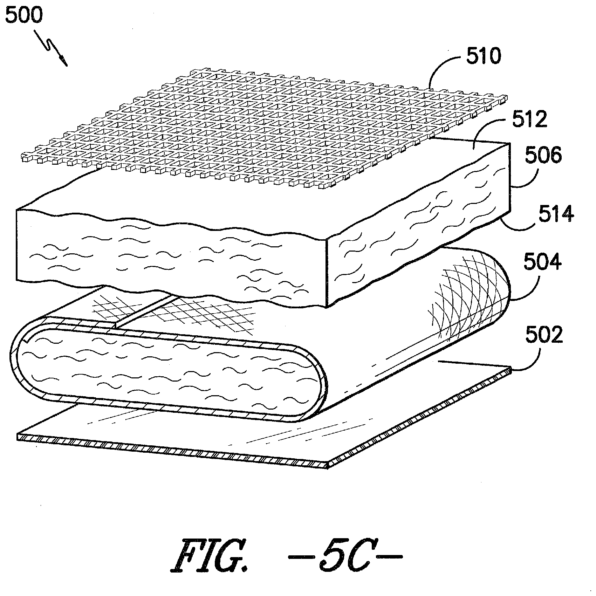

FIG. 5C illustrates a four-layer embodiment of the compression dressing 500 of the present invention wherein the pouch configuration of absorptive reservoir layer 504 is combined with additional layers comprising the compression dressing 500. The additional layers of the compression dressing 500 are similar to those described previously in FIGS. 1-4. In the instance wherein the absorptive reservoir layer 504 is provided in the shape of a tea bag or similar structure, the absorptive reservoir layer **504** may or may not be coextensive with the additional layers comprising the compression dressing **500.**

The following examples further illustrate the present compression dressing but are not to be construed as limiting the invention as defined in the claims appended hereto. All parts and percents given in these examples are by weight unless otherwise indicated.

### *Sample Creation and Evaluation*

### *A. Substrate Description*

Example 1, an example of a moisture transport fabric layer as described herein, was a double jersey knit (circular knit), multi-polymer fabric sold by Milliken & Company. The fabric was single layer of fabric comprised of 65% continuous filament polyamide yarn (nylon 6,6), 20% continuous filament polyester yarn, and 15% continuous filament spandex yarn. The polyamide yarn was comprised of 2 plies of 40 denier / 34 filament count nylon 6 fiber that was exposed to a texturing process prior to knitting. The polyester yarn was comprised of single ply 70 denier / 34 filament count fiber that was exposed to a texturing process prior to knitting. The spandex yarn was comprised of 55 denier / 1 filament count fiber. The fabric was knitted in such as manner as to give a distinct nylon side and a distinct polyester side. The polyester side of the fabric was exposed to a face-finishing process known as sanding.

The fabric was passed through an aqueous bath containing an antimicrobial formulation comprised of 15% AlphaSan® RC 2000 (antimicrobial agent, 10% Ag) and 4% Witcobond® UCX-281F (polyurethane binder). The coated fabric was subsequently passed through squeeze rollers to achieve a wet pick-up of 85%. The fabric was then dried in a tenter frame to remove excess liquid.

Example 1 was evaluated for surface energy characteristics and for tensile and elongation / compression properties, according to the test procedures described herein.

Example 1A was the same as Example 1, except that the spandex fiber of Example 1A was 140/1 yarn (rather than 55/1 yarn).

Example 2A was comprised of hydrophilic polyurethane foam, available from Rynel® of Wiscasset, Maine.

Example 2B was comprised of 30W hydrophilic polyurethane foam, available from Filtrona of Richmond, Virginia.

### *B. Test Procedures*

Surface Energy Test

Surface energy was determined by t/he Fowkes method using water and diiodomethane as probe liquids. The surface properties of these liquids are as follows:

| Liquids | Overall Surface Tension (mN/m) | Dispersive Component. (mN/m) | Polar Component. (mN/m) | Surface Polarity (%) |
|---|---|---|---|---|
| Water | 72.8 | 26.4 | 46.4 | 63.7 |
| Diiodomethane | 50.8 | 50.8 | 0.0 | 0.0 |

Contact angles were determined by the sessile drop method. In order to obtain proper surface energy values on the surfaces, care was taken to measure initial spreading angles and not angles which were influenced (diminished) by the liquids soaking into the samples. To achieve this goal, high speed image analysis was utilized whereby images were taken of drops of liquids placed on the surface of each material at a rate of 360 times per second. Using small drops (e.g. 1.0 microliter), it was determined that the drops did in fact spread to characteristic angles, before soaking into the sample significantly, in the time frame of 0.1 seconds after droplet placement for these liquids. Thus, the contact angle was used that measured at 0.1 seconds for each probe liquid on each surface as the characteristic contact angle for surface energy determination. By 0.2 seconds and beyond, some drops (particularly of diiodomethane were not only spread, but were also significantly soaking into the samples).

Tensile Test:

The effective pressure of compression bandages was quantified by measuring the tension of bandages using a tensile test. Tension is used to quantify compression instead of pressure since the pressure exerted on a body part (e.g. a leg or other limb) is a function of body part radius (EQ1.2). Textile samples were prepared in 2.54-10.16 cm (1" to 4") widths and 20.32 cm (8") lengths using a punch where needed. The samples were tested in either the machine direction or transverse direction of the textile.

Samples were mechanically tested for tensile strength, modulus, and recovery using a MTS Sintech 10/G mechanical tester with an MTS 25kN load cell. The samples were held in 3" wide hydraulic grips. Steel plates were used to hold samples wider than the 3" hydraulic grips. No slippage was observed during testing. Thus, the samples experienced uniform stress across their width. A 5" sample span between the grips was used. The MTS mechanical testing machine separated the hydraulic grips at a rate of 20.3 cm/min (8 inches/min), thereby stretching the samples. Samples were elongated multiple times and the force supported by the textile was measured in both extensional and relaxational directions.

### C. Test Results

Surface Energy Test:

Examples 1 and Examples 2A and 2B were tested for surface energy properties. The results are provided in Table 1 below.

**Table 1**

| Surface Energy Test | | | |
|---|---|---|---|
| Sample | Contact Angle of Water (degrees) | Contact Angle of Diiodomethane (degrees) | Surface Energy (mJ/m$^2$) |
| Example 1 - Polyester Side of Fabric | 98.1 ± 0.6 | 65.3 ± 0.6 | 26.14 |
| Example 1 - Nylon Side of Fabric | 94.8 ± 0.7 | 64.3 ± 0.8 | 27.19 |
| Example 2A | 66.6 ± 0.8 | 35.7 ± 0.4 | 47.8 |
| Example 2B | 85.9 ± 0.6 | 52.1 ± 0.3 | 35.0 |

Tensile Test:

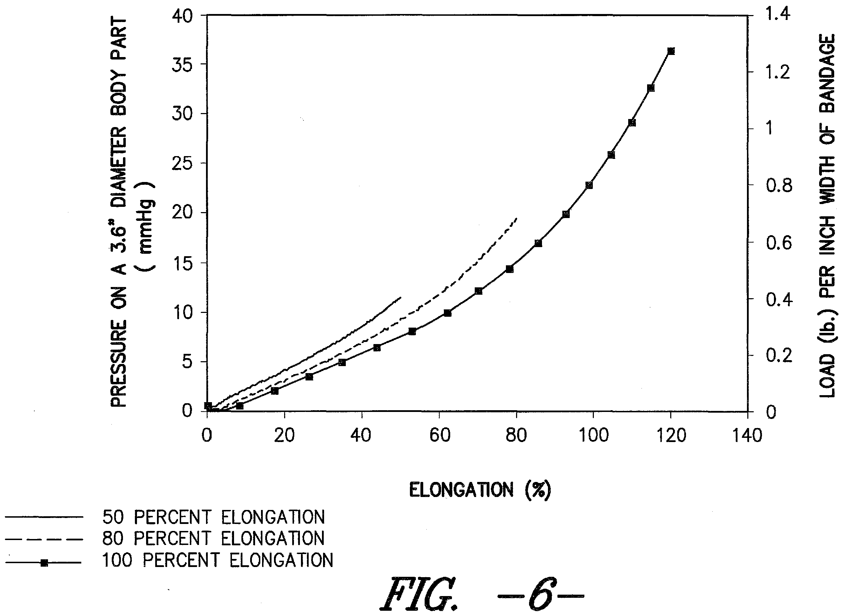

Example 1 was tested using tensile testing after being stretched once. As seen in Tables 2A, 2B and 2C and in **FIG. 6,** the amount of force exerted by the compression dressing depends on the elongation of the dressing. Preferably, the dressing will exert 0.89-71.33 kg/m (0.05-4 lbf/in) width of dressing at an elongation of 25-99% of maximum stretch. In general, this range allows for a clinician to adjust the level of compression per a specific patient's needs. Test results are provided in Tables 2A, 2B and 2C below.

**Table 2A**

| Elongation (%) | Load | | Pressure (mmHg at 9.14 cm (3.6 inch) diameter of curvature) |
|---|---|---|---|
| | (kg/m width) | (lbf/in width) | |
| -0.017 | 0.3805 | 0.021333 | 0.62635 |
| 1.176 | 0.2854 | 0.016 | 0.469762 |
| 3.84 | 0.6242 | 0.035 | 1.027605 |
| 6.505 | 0.9987 | 0.056 | 1.644169 |
| 9.17 | 1.3376 | 0.075 | 2.202012 |
| 11.834 | 1.6467 | 0.092333 | 2.710921 |
| 14.498 | 1.8726 | 0.105 | 3.082816 |
| 17.165 | 2.1877 | 0.122667 | 3.601512 |
| 19.829 | 2.5325 | 0.142 | 4.169142 |

(continued)

| Tensile Test - 50% Elongation | | | |
|---|---|---|---|
| Elongation (%) | Load | | Pressure (mmHg at 9.14 cm (3.6 inch) diameter of curvature) |
| | (kg/m width) | (lbf/in width) | |
| 22.493 | 2.8179 | 0.158 | 4.638904 |
| 25.157 | 3.1330 | 0.175667 | 5.1576 |
| 27.824 | 3.4956 | 0.196 | 5.75459 |
| 30.487 | 3.8344 | 0.215 | 6.312433 |
| 33.153 | 4.1495 | 0.232667 | 6.831129 |
| 35.82 | 4.5419 | 0.254667 | 7.477053 |
| 38.481 | 4.9699 | 0.278667 | 8.181696 |
| 41.146 | 5.4098 | 0.303333 | 8.905913 |
| 43.813 | 5.8141 | 0.326 | 9.57141 |
| 46.478 | 6.3134 | 0.354 | 10.39349 |
| 49.141 | 6.7890 | 0.380667 | 11.17643 |
| 50.143 | 6.9377 | 0.389 | 11.4211 |

**Table 2B**

| Tensile Tes - 80% Elongation | | | |
|---|---|---|---|
| Elongation (%) | Load | | Pressure (mmHg at 9.14 cm (3.6 inch) diameter of curvature) |
| | (kg/m width) | (lbf/in width) | |
| 0.124 | 0.5648 | 0.031667 | 0.929738 |
| 5.461 | 0.4221 | 0.023667 | 0.694857 |
| 10.789 | 0.9452 | 0.053 | 1.556088 |
| 16.118 | 1.5457 | 0.086667 | 2.544547 |
| 21.45 | 2.0629 | 0.115667 | 3.395991 |
| 26.778 | 2.6098 | 0.146333 | 4.296369 |
| 32.108 | 3.1983 | 0.179333 | 5.265254 |
| 37.437 | 3.8879 | 0.218 | 6.400514 |
| 42.768 | 4.5478 | 0.255 | 7.486839 |
| 48.096 | 5.2671 | 0.295333 | 8.671032 |
| 53.426 | 6.0757 | 0.340667 | 10.00203 |
| 58.757 | 6.9852 | 0.391667 | 11.49939 |
| 64.084 | 7.9542 | 0.446 | 13.09463 |
| 69.416 | 9.0124 | 0.505333 | 14.83666 |
| 74.745 | 10.3797 | 0.582 | 17.08761 |
| 80.008 | 11.7530 | 0.659 | 19.34834 |

**Table 2C**

| Tensile Test - 120% Elongation | | | |
|---|---|---|---|
| Elongation (%) | Load | | Pressure (mmHg at 9.14 cm (3.6 inch diameter) of curvature) |
| | (kg/m width) | (lbf/in width) | |
| 0 | 0.1367 | 0.007667 | 0.225095 |
| 1.767 | 0.0535 | 0.003 | 0.08808 |
| 7.094 | 0.3210 | 0.018 | 0.528483 |
| 12.425 | 0.8263 | 0.046333 | 1.360354 |
| 17.754 | 1.3970 | 0.078333 | 2.299879 |
| 23.082 | 1.8429 | 0.103333 | 3.033883 |
| 28.415 | 2.4136 | 0.135333 | 3.973407 |
| 33.742 | 2.8654 | 0.160667 | 4.717198 |
| 39.072 | 3.4064 | 0.191 | 5.607789 |
| 44.402 | 3.9593 | 0.222 | 6.517954 |
| 49.731 | 4.4824 | 0.251333 | 7.379185 |
| 55.061 | 5.1245 | 0.287333 | 8.436151 |
| 60.391 | 5.8497 | 0.328 | 9.63013 |
| 65.722 | 6.5810 | 0.369 | 10.8339 |
| 71.049 | 7.4133 | 0.415667 | 12.20404 |
| 76.381 | 8.4120 | 0.471667 | 13.84821 |
| 81.71 | 9.4702 | 0.531 | 15.59024 |
| 87.038 | 10.6413 | 0.596667 | 17.51823 |
| 92.37 | 11.9967 | 0.672667 | 19.7496 |
| 97.698 | 13.5305 | 0.758667 | 22.27457 |
| 101.962 | 14.8027 | 0.83 | 24.36893 |
| 107.295 | 16.7110 | 0.937 | 27.51046 |
| 112.622 | 18.7739 | 1.052667 | 30.90646 |
| 117.952 | 21.1043 | 1.183333 | 34.74285 |
| 120.016 | 22.0733 | 1.237667 | 36.33808 |

Elasticity Variability:

The spandex content of the moisture transport fabric layer of the present invention was varied. Example 1, described in detail above, contained 55/1 ROICA® spandex/elastane fiber in the jersey knit portion of the construction. Example 1A was the same as Example 1, except that it contained more spandex content. In order to this increase in spandex content for Example 1A, a 140/1 ROICA® spandex/elastane fiber was used to replace every third feed of 55/1 ROICA® spandex/elastane fiber. Inserting this larger denier spandex in a portion of the jersey knit construction increased the total spandex content of the fabric by 51 %. Alternate means of altering the elastic resistive force of the fabric under elongation include changing the number or size of spandex yarns in a portion of the jersey knit fabric, decreasing the portion of the jersey knit loops (feeds) which contain spandex fiber, changing the loop size and construction of the fabric, and/or using a different elastomeric material in the final fabric construction.

The increased spandex content fabric (Example 1A) was compared to the normal spandex fabric (Example 1) under 30% transverse heatset in the greige form.

Both fabrics were stretched to 100% elongation as described above. Test results are provided in Table 3 below.

**Table 3**

| Elasticity Variability Test | |
|---|---|
| Sample | Force per m (inch) width at 100% elongation (m/kg (Lbf/in)) |
| Example 1 | 9.798 kg/m (0.549 lbf/in) |
| Example 1A | 10.88 kg/m (0.61 lbf/in) |

These and other modifications and variations to the present invention may be practiced by those of ordinary skill in the art, without departing from the spirit and scope of the present invention. Furthermore, those of ordinary skill in the art will appreciate that the foregoing description is by way of example only, and is not intended to limit the scope of the invention described in the appended claims.

**Claims**

1. A compression dressing which

   (i) is at least 5.1 cm (2 inches) wide and 91.4 cm (36 inches) in length;
   (ii) provides one-way, active fluid management of fluid away from the skin and into the absorptive reservoir layer,
   (iii) comprises the following sequential layers:

      (a) optionally, a skin-contacting layer comprised of fabric, foam, alginate, hydrogel, hydrocolloid, polymeric film, medicinal agents, and combinations thereof,
      (b) at least one layer of moisture transport fabric which

         - is comprised of elastic fiber,
         - exhibits a sufficient amount of elasticity to apply a compressive force to a body part when applied thereto;
         - is a jersey knit fabric comprised of 100% synthetic fiber which is a blend of 40-80% nylon fiber, 10-50% polyester fiber and 5-25% spandex fiber, and
         - is comprised of hydrophobic fiber on the skin-facing surface of the fabric and hydrophilic fiber on the non skin-facing surface of the fabric,

      (c) at least one absorptive reservoir layer selected from textile substrates, foam, hydrogel, hydrocolloid, superabsorbent polymers, silica gel, water swelling polymers, polysaccharides, proteinaceous materials, and combinations thereof; and
      (d) optionally, at least one layer of elastic fabric comprising the outermost layer of the compression dressing; and

   (iv) exhibits a tensile strength of 0.89-25.67 kg/m width (0.05-2.0 lbf/inch width) at 50% elongation, determined using a MTS Sintech 10/G mechanical tester with an MTS 25kN load cell and stretching at 20.3 cm/min (8 inches/min).

2. The compression dressing of claim 1, which comprises the skin-contacting layer (a).

3. The compression dressing of claim 1 or 2, which comprises the at least one layer of elastic fabric (d).

4. The compression dressing of any of claims 1-3, wherein layer (b) further contains an antimicrobial agent, wherein the antimicrobial agent is a silver-ion containing compound.

5. The compression dressing of claim 4, wherein the silver-ion containing compound is a silver zirconium phosphate compound.

6. The compression dressing of claim 4, wherein layer (b) further contains a binding agent.

7.  The compression dressing of claim 6, wherein the binding agent is a polyurethane-based compound.

8.  The compression dressing of claim 3, wherein layer (d) is selected from a nonwoven fabric, a knit fabric, and a woven fabric.

9.  The compression dressing of any of claims 1-3, wherein the layers comprising the compression dressing are affixed to each other.

10. The compression dressing of any of claims 1-3, wherein the tensile strength is 1.25-17.83 kg/m width (0.07-1.0 lbf/inch width) at 50% elongation.

11. The compression dressing of any of claims 1-3, wherein the layers comprising the dressing are coextensive with one another.

**Patentansprüche**

1.  Kompressionsverband, der

    (i) mindestens 5,1 cm (2 Zoll) breit und 91,4 cm (36 Zoll) lang ist;
    (ii) ein unidirektionales aktives Fluid-Management von Fluid weg von der Haut in die absorptionsfähige Reservoirschicht bereitstellt,
    (iii) die folgenden aufeinanderfolgenden Schichten umfasst:

    (a) optional eine mit der Haut in Kontakt stehende Schicht, die aus Stoff, Schaum, Alginat, Hydrogel, Hydrocolloid, Polymerfilm, medizinischen Mitteln und Kombinationen hiervon umfasst,
    (b) mindestens eine Schicht aus Feuchtigkeitstransportierendem Stoff, der

    - eine elastische Faser umfasst,
    - ein ausreichendes Maß an Elastizität aufweist, um eine Druckkraft auf ein Körperteil auszuüben, wenn er hierauf aufgebracht wird;
    - ein Jersey-Strickstoff ist, der eine 100 % synthetische Faser umfasst, die eine Mischung aus 40-80 % Nylonfaser, 10-50 % Polyesterfaser und 5-25 % Elastanfaser ist, und
    - auf der der Haut zugewandten Oberfläche des Stoffs eine hydrophobe Faser und auf der der Haut nicht zugewandten Oberfläche des Stoffs eine hydrophile Faser umfasst,

    (c) mindestens eine absorptionsfähige Reservoirschicht, ausgewählt aus Textilsubstraten, Schaum, Hydrogel, Hydrocolloid, superabsorbierenden Polymeren, Silikagel, Wasser-quellenden Polymeren, Polysachariden, proteinartigen Materialien und Kombinationen hiervon; und
    (d) optional mindestens eine Schicht aus elastischem Stoff, umfassend die äußerste Schicht des Kompressionsverbands; und

    (iv) der eine Zugfestigkeit von 0,89-25,67 kg/m Breite (0,05-2,0 Pfund/Zoll Breite) bei 50 % Dehnung aufweist, bestimmt unter Verwendung eines mechanischen Testgeräts MTS Sintech 10/G mit einer MTS 25kN-Kraftmesszelle und bei einer Streckung von 20,3 cm/min (8 Zoll/min).

2.  Kompressionsverband gemäß Anspruch 1, der die Hautkontaktschicht (a) umfasst.

3.  Kompressionsverband gemäß Anspruch 1 oder 2, der zumindest eine Schicht aus elastischem Stoff (d) umfasst.

4.  Kompressionsverband gemäß irgendeinem der Ansprüche 1-3, worin die Schicht (b) ferner ein antimikrobielles Mittel umfasst, worin das antimikrobielle Mittel eine silberionenhaltige Verbindung ist.

5.  Kompressionsverband gemäß Anspruch 4, worin die silberionenhaltige Verbindung eine Silber-Zirkonium-Phosphatverbindung ist.

6.  Kompressionsverband gemäß Anspruch 4, worin die Schicht (b) ferner ein Bindemittel enthält.

7. Kompressionsverband gemäß Anspruch 6, worin das Bindemittel eine Verbindung auf Polyurethanbasis ist.

8. Kompressionsverband gemäß Anspruch 3, worin die Schicht (d) ausgewählt ist aus einem Vliesstoff, einem Strickstoff und einem Webstoff.

9. Kompressionsverband gemäß irgendeinem der Ansprüche 1-3, worin die Schichten, die der Kompressionsverband umfasst, miteinander verbunden sind.

10. Kompressionsverband gemäß irgendeinem der Ansprüche 1-3, worin die Zugfestigkeit 1,25-17,83 kg/m Breite (0,07-1,0 Pfund/Zoll Breite) bei 50 % Dehnung beträgt.

11. Kompressionsverband gemäß irgendeinem der Ansprüche 1-3, worin die Schichten, die der Verband umfasst, koextensiv zueinander sind.

**Revendications**

1. Pansement compresse qui

(i) est d'au moins 5,1 cm (2 pouces) de large et 91,4 cm (36 pouces) de long;
(ii) fournit une gestion de fluide actif unidirectionnel qui éloigne le fluide de la peau et dans la couche de réservoir d'absorption,
(iii) comprend les couches séquentielles suivantes :

(a) facultativement, une couche de contact avec la peau composée de tissu, de mousse, d'alginate, d'hydrogel, d'hydrocolloïde, de film polymère, d'agents médicinaux et de combinaisons de ceux-ci,
(b) au moins une couche de tissu de transport d'humidité qui

- est composée de fibre élastique,
- présente une quantité suffisante d'élasticité pour appliquer une force de compression sur une partie de corps lorsqu'il est appliqué dessus ;
- est un tissu en jersey composé de 100 % de fibre synthétique qui est un mélange de 40 à 80 % de fibre de nylon, de 10 à 50 % de fibre de polyester et de 5 à 25 % de fibre d'élasthanne, et
- est composée de fibre hydrophobe sur la surface tournée vers la peau du tissu et de fibre hydrophile sur la surface non tournée vers la peau du tissu,

(c) au moins une couche de réservoir d'absorption sélectionnée parmi des substrats textiles, une mousse, un hydrogel, un hydrocolloïde, des polymères superabsorbants, un gel de silice, des polymères gonflant dans l'eau, des polysaccharides, des matériaux protéiques et des combinaisons de ceux-ci ; et
(d) facultativement, au moins une couche de tissu élastique comprenant la couche la plus extérieure du pansement compresse ; et

(iv) présente une résistance à la traction de 0,89 à 25,67 kg/m de large (0,05 à 2,0 livres-force/pouce de large) à un allongement de 50 %, déterminée en utilisant un testeur mécanique 10/G MTS Sintech avec une cellule de charge de 25 kN de MTS et un étirement à 20,3 cm/min (8 pouces/min).

2. Pansement compresse selon la revendication 1, qui comprend la couche de contact avec la peau (a).

3. Pansement compresse selon la revendication 1 ou 2, qui comprend l'au moins une couche de tissu élastique (d).

4. Pansement compresse selon l'une quelconque des revendications 1 à 3, dans lequel la couche (b) contient en outre un agent antimicrobien, dans lequel l'agent antimicrobien est un composé contenant des ions argent.

5. Pansement compresse selon la revendication 4, dans lequel le composé contenant des ions argent est un composé de phosphate d'argent et de zirconium.

6. Pansement compresse selon la revendication 4, dans lequel la couche (b) contient en outre un agent de liaison.

**7.** Pansement compresse selon la revendication 6, dans lequel l'agent de liaison est un composé à base de polyuréthane.

**8.** Pansement compresse selon la revendication 3, dans lequel la couche (d) est sélectionnée parmi un tissu non tissé, un tissu tricoté et un tissu tissé.

**9.** Pansement compresse selon l'une quelconque des revendications 1-3, dans lequel les couches comprenant le pansement compresse sont fixées entre elles.

**10.** Pansement compresse selon l'une quelconque des revendications 1-3, dans lequel la résistance à la traction est de 1,25 à 17,83 kg/m de large (0,07 à 1,0 livre-force/pouce de large) à un allongement de 50 %.

**11.** Pansement compresse selon l'une quelconque des revendications 1-3, dans lequel les couches comprenant le pansement sont coextensives entre elles.

100

110

106

104

102

## FIG. -1-

100

110

112

106

114

104

102

## FIG. -1A-

200

202

204

202

204

204

202

## FIG. -2-

300

310

312

306

309

314

304

302

## FIG. -3-

FIG. −4−

504

505

507

509

## FIG. -5A-

504

509

507

## FIG. -5B-

FIG. −5C−

FIG. -6-

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5939339 A
- US 6573419 B
- EP 1709947 A
- US 2010030170 A
- US 2010030171 A
- US 2010030178 A
- US 6584668 B
- US 6821936 B
- US 6946433 B
- US 20040106340 A
- US 20040106341 A